# EUROPEAN PATENT APPLICATION

(11) **EP 2 042 195 A1**
(43) Date of publication of application: **01.04.2009**
(21) Application number: 07790771.5
(22) Date of filing: 13.07.2007
(51) Int. Cl.: A61K 47/48, A61P 9/00, A61P 35/00, C08G 81/00

(54) **POLYMER CONJUGATE OF COMBRETASTATIN**

(30) Priority: 19.07.2006 JP 2006196503
(71) Applicant: Nippon Kayaku Kabushiki Kaisha, Tokyo 102-8172 (JP)
(72) Inventor: KITAGAWA, Masayuki, Tokyo 115-8588 (JP); ISHIKAWA, Keizou, Tokyo 115-8588 (JP); MASUDA, Akira, Tokyo 115-8588 (JP); TAKASHIO, Kazutoshi, Tokyo 115-8588 (JP)
(74) Representative: Wablat, Wolfgang
(86) International application number: PCT/JP2007/063990
(87) International publication number: WO 2008/010463

(57) **Abstract**

A novel derivative of combretastatins which has water solubility and is capable of releasing the drug independent of biological enzymes likely to cause individual differences and whose effective therapeutic effect can be expected has been demanded. A high-molecular weight conjugate of combretastatins, **characterized by** having a structure in which a hydroxyl group of a combretastatin is linked via an ester bond to a carboxylic acid group of the polymer moiety in a block copolymer of a polyethylene glycol moiety with the polymer moiety having two or more carboxylic acid groups such as polyaspartic acid or polyglutamic acid is provided.

## Description

### Technical Field

The present invention relates to a high-molecular weight conjugate of combretastatins comprising a block copolymer of a polyethylene glycol moiety and the polymer moiety having two or more carboxylic acids, in which a carboxylic acid group of the polymer moiety is linked to a hydroxyl group of combretastatins via an ester bond, a method for manufacturing the same, and the use thereof.

### Background Art

Combretastatin was isolated from the native African tree Combretum caffrum and the like in the 1980's, and was verified to have tubulin polymerization inhibitory activity. In particular, the compound has blood flow inhibitory activity by causing morphological changes of the vascular endothelial cells. Therefore, the compound is expected to be used as a therapeutic agent for diseases associated with neovascularization, such as solid cancers and rheumatoid arthritis. A method for manufacturing combretastatins is described in Non-Patent Document 1. However, combretastatins are generally poorly water-soluble, and therefore research has been conducted to impart water-solubility to the compounds or to allow the compounds to exert their effect more efficiently in the affected areas.

Heretofore, a method for manufacturing a phosphoric acid ester-type prodrug of combretastatins has been described in Patent Document 1. Furthermore, Non-Patent Document 2 describes a combretastatin derivative having an amino group, and an amino acid-conjugated prodrug for allowing the derivative to selectively exert the medical effects in the affected areas.

In the meantime, research has also been conducted on the use of a macromolecule as a carrier for the purpose of imparting water-solubility to poorly water-soluble anticancer agents, or accumulating the anticancer agents in the affected areas. For example, Patent Document 2 and Patent Document 3 describe, as a prodrug, high-molecular weight derivatives of poorly water-soluble anticancer agents bound with polyethylene glycol. However, conjugates of combretastatins are not described. Furthermore, in these high-molecular weight conjugates of the poorly water-soluble anticancer agents, only one or two molecules of the anticancer agents can be bound to one molecule of polyethylene glycol for structural reason. Therefore, a large amount of polymer is necessary in order to administer an effective amount of the drug.

Patent Document 4 describes a molecule in which a drug is bound to a block copolymer of polyethylene glycol and polyaspartic acid, which forms micelles and has water-solubility. Patent Document 5 describes a high-molecular weight carrier in which a hydrophobic substance is bound to a carboxylic acid group in a side chain of a block copolymer of a polyethylene glycol and a poly (acidic amino acid), and which can serve as a high-molecular weight carrier for drugs. Patent Document 6 describes a high-molecular weight derivative of camptothecins in which a carboxylic acid group in a side chain of a block copolymer of a polyethylene glycol and polyglutamic acid is linked to a phenolic hydroxyl group of the camptothecins. However, Patent Document 4, Patent Document 5 and Patent Document 6 do not describe conjugates of combretastatins.
Patent Document 1: WO 02/06279
Patent Document 2: WO 93/24476
Patent Document 3: Japanese Patent Application Laid-Open (KOHYO) No. 10-513187
Patent Document 4: Japanese Patent No. 2694923
Patent Document 5: Japanese Patent No. 3268913
Patent Document 6: WO 04/39869
Non-Patent Document 1: J. Org. Chem., 66, 8135-8138 (2001)
Non-Patent Document 2: Anti-Cancer Drug Design, 14, 539-548 (1999)

### Disclosure of the Invention

### Problems to be Solved by the Invention

The prodrug of combretastatins described in Non-Patent Document 2 has an increased solubility in water as compared with combretastatin A-4. However, since the release of combretastatin A-4 depends on an endogenous aminopeptidase in the blood of the subject to be administered, there is concern that individual differences may be caused to the effect obtained by the released drug.

The phosphoric acid ester-type prodrug of combretastatins of Patent Document 1 has an increased solubility in water as compared with combretastatin A-4. However, there is concern that the phosphoric acid ester is hydrolyzed immediately after administration into the body, and thus it is uncertain whether the prodrug would be delivered to the affected areas to allow combretastatin A-4 to efficiently exert the effect. The bond between a polyethylene glycol moiety and a drug described in Patent Document 2 or Patent Document 3 is also cleavable by hydrolyzing enzymes in the body, by which the delivery and release of the drug is controlled. However, the hydrolyzying enzymes in the body are thought to vary widely among different species, even among individuals within the same species. Therefore, with regard to the drug conjugates described in these documents, there is concern that individual differences in the effect by the released drug may be caused because the cleavage of the bond to drug depends on the hydrolyzing enzymes in the body.

Furthermore, in the case of the adriamycin conjugate described in Patent Document 5, a block copolymer is bound to adriamycin via an amide bond. However, since the amide bond is a chemically stable form of bond, the release of the drug by hydrolysis is slow, and the effect is questionable.

Combretastatin compounds such as combretastatin A-4 are useful anticancer agents, but are poorly soluble. Therefore, there is a demand for novel derivatives which have water-solubility and are excellent in anticancer activity.

### Means for Solving the Problems

As a result of intensive studies for solving the problem described above, the present inventors have found that a high-molecular weight conjugate of combretastatins comprising a block copolymer of a polyethylene glycol moiety and a polymer moiety having two or more carboxylic acid groups, in which a carboxylic group of the polymer moiety is linked to a hydroxyl group of the combretastatins via an ester bond, releases the combretastatins without depending on a hydrolyzing enzyme, and thus have completed the present invention.

Accordingly, the present invention relates to the following 1) to 15).
(1) A high-molecular weight conjugate of combretastatins comprising a block copolymer of a polyethylene glycol moiety and a polymer moiety having two or more carboxylic acids, in which a carboxylic acid group of the polymer moiety having two or more carboxylic acid is linked to a hydroxyl group of combretastatins via an ester bond.
(2) The high-molecular weight conjugate of combretastatins according to (1), wherein the polymer moiety having carboxylic acid groups is a polymer having succinic acid monoamide moieties.
(3) The high-molecular weight conjugate of combretastatins according to (2), wherein the polymer having succinic acid monoamide moieties is polyaspartic acid.
(4) The high-molecular weight conjugate of combretastatins according to any one of (1) to (3), which is a compound represented by general formula (I): 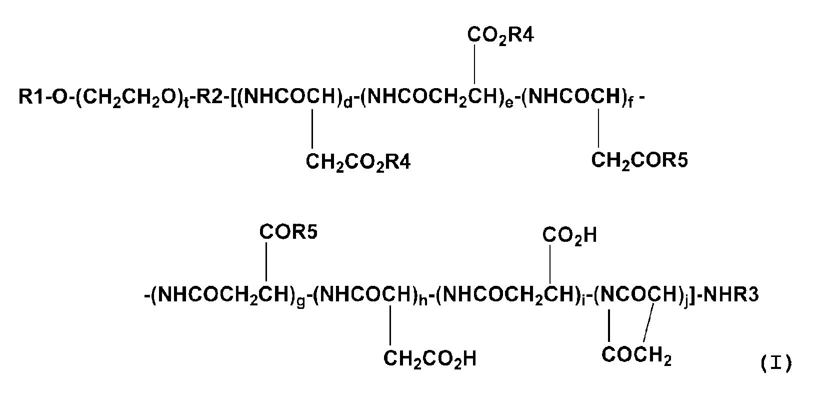 wherein R1 represents a hydrogen atom or a (C1-C6) alkyl group; R2 represents a linking group; R3 represents a hydrogen atom or a (C1-C6) acyl group; R4 represents a residue of a hydroxyl group of the combretastatins; R5 represents a group selected from the group consisting of a (C1-C30) alkoxy group, a (C7-C30) aralkyloxy group, a (C1-C30) alkylamino group, a di(C1-C30) alkylamino group, an amino acid with a protected carboxyl group and -N(R6)CONH(R7) wherein R6 and R7, which may be identical or different, each represent a (C3-C6) cyclic alkyl group, or a (C1-C5) alkyl group which may be substituted with a tertiary amino group; t represents an integer from 5 to 11500; d, e, f, g, h, i or j each independently represent an integer from 0 to 200; with the proviso that d+e represent an integer from 1 to 200; and d+e+f+g+h+i+j represent an integer from 3 to 200; and the respective constituent units of the polyaspartic acid are bound in any order.
(5) The high-molecular weight conjugate of combretastatins according to (4), wherein R1 is a (C1-C6) alkyl group; R2 is a (C2-C6) alkylene group; R3 is a (C1-C6) acyl group; t is an integer from 8 to 2300; and d, e, f, g, h, i or j are each independently an integer from 0 to 100; with the proviso that d+e is an integer from 1 to 100, and d+e+f+g+h+i+j is an integer from 6 to 100.
(6) The high-molecular weight conjugate of combretastatins according to (4) or (5), wherein R1 is a (C1-C3) alkyl group; R2 is a (C2-C4) alkylene group; R3 is a (C1-C3) acyl group; t is an integer from 100 to 300; and d, e, f, g, h, i or j each independently is an integer from 0 to 90; with the proviso that d+e is an integer from 1 to 90, and d+e+f+g+h+i+j is an integer from 15 to 90.
(7) The high-molecular weight conjugate of combretastatins according to (1), wherein the polymer moiety having carboxylic acid groups is polyglutamic acid.
(8) The high-molecular weight conjugate of combretastatins according to (1) to (7), which is a compound represented by general formula (II): 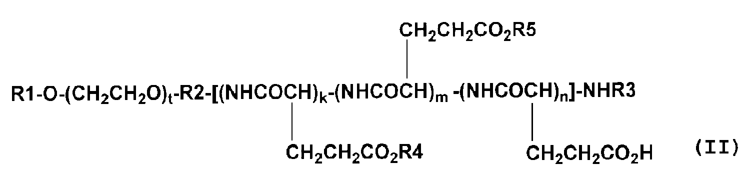 wherein R1 represents a hydrogen atom or a (C1-C6) alkyl group; R2 represents a linking group; R3 represents a hydrogen atom or a (C1-C6) acyl group; R4 represents a residue of a hydroxyl group of the combretastatins; R5 represents a group selected from the group consisting of a (C1-C30) alkoxy group, a (C7-C30) aralkyloxy group, a (C1-C30) alkylamino group, a di(C1-C30) alkylamino group, an amino acid with a protected carboxyl group and -N(R6)CONH(R7) wherein R6 and R7, which may be identical or different, each represent a (C3-C6) cyclic alkyl group, or a (C1-C5) alkyl group which may be substituted with a tertiary amino group; t represents an integer from 5 to 11500; k represents an integer from 1 to 200; m and n each independently represent an integer from 0 to 200; with the proviso that k+m+n represents an integer from 3 to 200; and the respective constituent units of the polyglutamic acid are bound in any order.
(9) The high-molecular weight conjugate of combretastatins according to (8), wherein R1 is a (C1-C6) alkyl group; R2 is a (C2-C6) alkylene group; R3 is a (C1-C6) acyl group; t is an integer from 8 to 2300; and k is an integer from 1 to 90; and m and n are each independently an integer from 0 to 90; with the proviso that k+m+n is an integer from 6 to 90.
(10) The high-molecular weight conjugate of combretastatins according to (8) or (9), wherein R1 is a (C1-C3) alkyl group; R2 is a (C2-C4) alkylene group; R3 is a (C1-C3) acyl group; t is an integer from 100 to 300; k is an integer from 3 to 60; and m and n are each independently an integer from 0 to 60; with the proviso that k+m+n is an integer from 6 to 60.
(11) The high-molecular weight conjugate of combretastatins according to any one of (1) to (10), wherein the combretastatin is combretastatin A-4.
(12) A high-molecular weight conjugate of combretastatins, obtained by, in a block copolymer of a polyethylene glycol moiety and a polymer moiety having two or more carboxylic acid groups, linking a carboxylic acid group of the polymer moiety to a hydroxyl group of the combretastatins via an ester bond using a dehydrating condensing agent in an organic solvent.
(13) A method for manufacturing a high-molecular weight conjugate of combretastatins according to any one of (1) to (11), the method comprising linking a carboxylic acid group of the polymer moiety to a hydroxyl group of the combretastatins via an ester bond using a dehydrating condensing agent in an organic solvent.
(14) An anticancer agent comprising the high-molecular weight conjugate of combretastatins according to (1) to (12), as an active ingredient.
(15) A vascular targeting agent comprising the high-molecular weight conjugate of combretastatins according to (1) to (12), as an active ingredient.

### Effect of the Invention

In the high-molecular weight conjugate of combretastatins of the present invention comprises a block copolymer of a polyethylene glycol moiety and a polymer moiety having two or more carboxylic acid groups, in which a carboxylic acid group of the polymer is linked to a hydroxyl group of the combretastatins via an ester bond. For this reason, the high-molecular weight conjugate of combretastatins of the present invention is capable of releasing the drug without depending on hydrolytic enzymes in the living body, and exhibits an effective therapeutic effect which is hardly affected by individual differences.

### Best Mode for Carrying Out the Invention

The high-molecular weight conjugate of combretastatins of the present invention comprises a block copolymer having a polyethylene glycol moiety and a polymer moiety having two or more carboxylic acid groups, in which a carboxylic acid group of the polymer moiety is linked to a hydroxyl group of the combretastatins via an ester bond. In the present invention, polymers having two or more carboxylic acid groups in the present invention are not particularly limited, but for example, include a polymer having two or more succinic acid monoamide moieties, polyglutamic acid and the like.

According to the present invention, the term succinic acid monoamide moiety means the structure -HNCO-C-C-CO₂H. Examples include succinic acid monoamide (-HNCO-CH₂-CH₂-CO₂H), a structure in which one of the two carboxylic acid groups of aspartic acid is amidated (-HNCO-CH(-NH-)-CH₂-CO₂H or -HNCO-CH₂-CH(-NH-)-CO₂H), or the like. These succinic acid monoamide moieties may constitute a polymer backbone, for example, as in the case of polyaspartic acid. Alternatively, the moieties may be bound to functional groups of the backbone polymer composed of a polyalcohol, such as dextran, a polyamine, such as polylysine, a polycarboxylic acid other than polyaspartic acid (for example, polylactic acid or the like).

The succinic acid monoamide moiety is thought to release a compound having a hydroxyl group as the moiety changes to a cyclized structure (succinic acid imide).

Examples of the polyethylene glycol moiety in the polymer of the high-molecular weight conjugate of combretastatins of the present invention include polyethylene glycol modified at both ends or at one end, and in the case where the both ends are modified, the modifying groups may be identical or different. Examples of the modifying group include a (C1-C6) alkyl group which may have substituents. Examples of the alkyl group of the (C1-C6) alkyl group which may have substituents may include the alkyl groups described below. Preferred may be a (C1-C4) alkyl group, and examples thereof include a methyl group, an ethyl group, a n-propyl group, a n-butyl group and the like. Examples of the substituents of the (C1-C6) alkyl group which may have the substituents include an amino group, a methylamino group, a dimethylamino group, an ethylamino group, a diethylamino group, and the like.

The molecular weight of the polyethylene glycol moiety is about from 300 to 500,000, preferably about from 500 to 100,000, more preferably about from 1000 to 50,000.

The molecular weight of the block copolymer of a polyethylene glycol moiety and a polymer moiety having two or more carboxylic acid groups according to the present invention is about 500 to 500,000, preferably about 600 to 100,000, more preferably about 800 to 80,000.

According to the present invention, the molecular weight refers to a weight average molecular weight determined by a GPC method.

In the high-molecular weight conjugate of combretastatins of the present invention, the amount of the combretastatins bound to the block copolymer of a polyethylene glycol moiety and a polymer moiety having two or more carboxylic acid groups is 1 to 100%, preferably 1 to 90%, and more preferably 2 to 60%, of the total number of carboxylic acid groups.

According to the present invention, the combretastatins are not particularly limited, provided that they are combretastatin skeleton compounds having a hydroxyl group and antitumor activity, that is, compounds having a stilbene structure having an oxygen functional group. Examples of the combretastatins include combretastatin A-1 (III), combretastatin A-4 (IV) and AC-7700 (V) represented by the following formulas, and the like. The hydroxyl group of the combretastatins includes a phenolic hydroxyl group as well as an alcoholic hydroxyl group, as shown by the following formulas, and, in addition, the position of substitution is not limited.

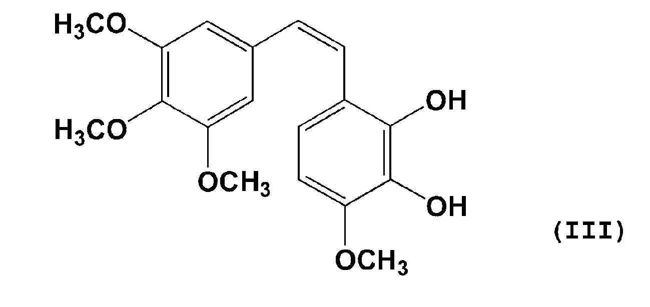

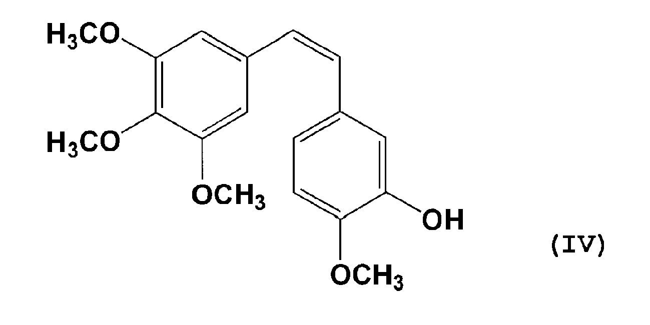

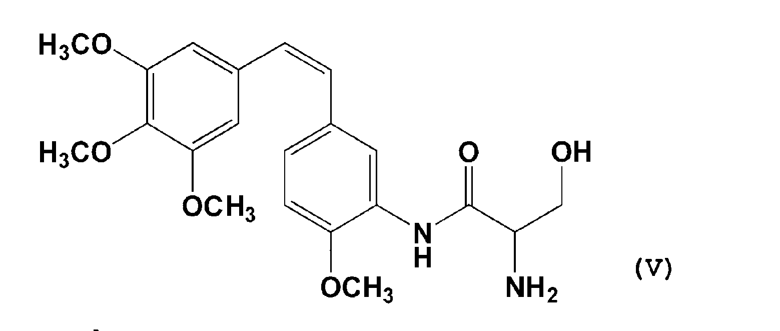

According to the present invention, the succinic acid monoamide moiety is preferably polyaspartic acid. As other examples of a polymer moiety having two or more carboxylic acid groups, polyglutamic acid is preferred.

Preferable high-molecular weight conjugates of combretastatins of the present invention may include a compound represented by the above general formula (I) containing polyaspartic acid [wherein R1 represents a hydrogen atom or a (C1-C6) alkyl group; R2 represents a linking group; R3 represents a hydrogen atom or a (C1-C6) acyl group; R4 represents the residue of a hydroxyl group of the combretastatins; R5 represents a group selected from the group consisting of a (C1-C30) alkoxy group, a (C7-C30) aralkyloxy group, a (C1-C30) alkylamino group, a di(C1-C30) alkylamino group, an amino acid with a protected carboxyl group and -N(R6)CONH(R7) wherein R6 and R7, which may be identical or different, each represent a (C3-C6) cyclic alkyl group or a (C1-C5) alkyl group which may be substituted with a tertiary amino group; t represents an integer from 5 to 11500; d, e, f, g, h, i or j each independently represent an integer from 0 to 200; with the proviso that d+e represent an integer from 1 to 200; and d+e+f+g+h+i+j represent an integer from 3 to 200; and the the respective constituent units of the polyaspartic acid are bound in any order], or a compound represented by the above general formula (II) containing polyglutamic acid [wherein R1 represents a hydrogen atom or a (C1-C6) alkyl group; R2 represents a linking group; R3 represents a hydrogen atom or a (C1-C6) acyl group; R4 represents a residue of a hydroxyl group of the combretastatins; R5 represents a group selected from the group consisting of a (C1-C30) alkoxy group, a (C7-C30) aralkyloxy group, a (C1-C30) alkylamino group, a di(C1-C30) alkylamino group, an amino acid with a protected carboxyl group and -N(R6)CONH(R7) wherein R6 and R7, which may be identical or different, each represent a (C3-C6) cyclic alkyl group or a (C1-C5) alkyl group which may be substituted with a tertiary amino group; t represents an integer from 5 to 11500; k represents an integer from 1 to 200; m and n each independently represent an integer from 0 to 200; with the proviso that k+m+n represents an integer from 3 to 200; and the respective constituent units of the polyglutamic acid are bound in any order].

Examples of the (C1-C6) alkyl group for R1 of the above general formula (I) or (II) include a straight-chain or branched alkyl group of carbon number of 1 to 6, and preferred is a straight-chain or branched (C1-C4) alkyl group, and particularly preferred is a straight-chain or branched (C1-C3) alkyl group. Examples of the straight-chain or branched (C1-C6) alkyl group include a methyl group, an ethyl group, a n-propyl group, an i-propyl group, a n-butyl group, a t-butyl group, a n-pentyl group, a n-hexyl group, and the like. Particularly preferred are a methyl group, an ethyl group, a n-propyl group and an i-propyl group. Among them, a methyl group is preferred.

Examples of the linking group represented by R2 of the above general formula (I) or (II) include, but are not particularly limited to, a (C2-C6) alkylene group, and preferred inter alia is a (C2-C4) alkylene group. Examples of the (C2-C4) alkylene group include an ethylene group, a trimethylene group, a butylene group and the like, and a trimethylene group is particularly preferred.

Examples of the (C1-C6) acyl group for R3 of the above general formula (I) or (II) include, but are not particularly limited to, a formyl group, an acetyl group, a propionyl group, a pivaloyl group and the like, an acetyl group is preferred.

With regard to the residue of a hydroxyl group of combretastatins for R4 of the above general formula (I) or (II), examples of the combretastatins include the aforementioned combretastatins. The combretastatins are not particularly limited provided that they have a hydroxyl group which is linked to a carboxylic acid group of the polymer moiety via an ester bond by a dehydrating condensing agent, and have antitumor activity. The preferable combretastatins include, for example, combretastatin A-4 shown above. R5 of the above general formula (I) or (II) represents a group selected from the group consisting of a (C1-C30) alkoxy group, a (C7-C30) aralkyloxy group, a (C1-C30) alkylamino group, a di(C1-C30) alkylamino group, an amino acid with a protected carboxyl group, and -N (R6) CONH (R7) wherein R6 and R7, which may be identical or different, are each a (C3-C6) cyclic alkyl group, or a (C1-C5) alkyl group which may be substituted with a tertiary amino group. R5 of the general formula (I) or (II) may be identical or different in one molecule, and may be single type or a mixed type in a polymer employed in the high-molecular weight conjugate of combretastatins.

Examples of the (C1-C30) alkoxy group include a straight-chain or branched (C1-C30) alkoxy group, and preferred is a straight-chain or branched (C1-C10) alkoxy group, including, for example, a methoxy group, an ethoxy group, a n-propoxy group, an i-propoxy group, a n-butoxy group, a t-butoxy group and the like. As the (C7-C30) aralkyloxy group, a straight-chain or branched (C7-C30) aralkyloxy group, or a straight-chained or branched (C7-C12) aralkyloxy group is preferred. For example, a 4-phenylbutoxy group and the like are mentioned.

Examples of the (C1-C30) alkylamino group or di(C1-C30) alkylamino group include a straight-chain or branched (C1-C30) alkylamino group or a di(C1-C30) alkylamino group, and preferred is straight-chain or branched (C1-C20) alkylamino group or a di (C1-C20) alkylamino group, including, for example, a methylamino group, an ethylamino group, a n-propylamino group, an i-propylamino group, a n-butylamino group, a t-butylamino group, a dimethylamino group, a diethylamino group, a dibutylamino group, and the like.

Examples of the amino acid with a protected carboxyl group include an amino acid usually used in peptide synthesis, in which the carboxyl group is protected. For example, a phenylalanine benzyl ester and the like are preferred.

Examples of the group -N (R6) CONH (R7) [wherein R6 and R7, which may be identical or different, are each a (C3-C6) cyclic alkyl group, or a (C1-C5) alkyl group which may be substituted with a tertiary amino group] for R5 of the general formula (I) or (II) is not particularly limited. The examples include a cyclohexylaminocarbonylcyclohexylamino group, an isopropylaminocarbonylisopropylamino group, and the like.

Polyaspartic acid, which is two or more succinic acid monoamide moieties in the high-molecular weight conjugate of combretastatins represented by the above general formula (I) of the present invention, includes constituent units of α-amino acid type, β-amino acid type, cyclized type and the like, but the polyaspartic acid in which all of the units are cyclized is not included. The linking order of these constituent units is not limited, and may be of block type or random type. Furthermore, each of the constituent units may be of L-type or D-type.

The total number of aspartic acids in the high-molecular weight conjugate of combretastatins represented by the above general formula (I), which is represented by d+e+f+g+h+i+j, is about from 3 to 200, preferably about from 6 to 100, particularly preferably from 15 to 90. The total number of aspartic acid can be freely changed, for example, according to the amounts of a polyethylene glycol moiety and polyaspartic acid fed to the reaction for producing the block copolymer.

The proportion of the number of aspartic acid bound to combretastatins (d+e) based on the total number of aspartic acids (d+e+f+g+h+i+j) is 1 to 100%, preferably 3 to 90%, more preferably 4 to 60%. Furthermore, the number of aspartic acid (d+e) is about 1 to 200, preferably about 1 to 100, particularly preferably about 1 to 90. The proportion of bound combretastatins can be changed, for example, according to the amounts a block copolymer and combretastatins fed to the reaction for their binding reaction and can be determined by an analysis of the reaction liquid, as described below.

The proportion of the α-amino acid type (d+f+h) based on the total number of aspartic acid (d+e+f+g+h+i+j) is 10 to 100%, preferably 20 t 100%. This proportion can be appropriately changed, for example, by selecting the deprotection conditions for the protecting group of polyaspartic acid and the like.

Polyglutamic acid in the high-molecular weight conjugate of combretastatins represented by the above general formula (II) of the present invention is of α-amino acid type, and may be of L-type or D-type.

The total number of glutamic acid in the high-molecular weight conjugate of combretastatins represented by the above general formula (II) of the present invention is represented by k+m+n, and is about 3 to 200, preferably about 6 to 90, particularly preferably about 6 to 60. The total number of glutamic acids can be freely changed, for example, according to the amounts of a polyethylene glycol moiety and polyglutamic acid fed to the reaction for producing the block copolymer.

The proportion of the number of the glutamic acids bound to combretastatins (k) based on the total number of glutamic acids (k+m+n) is 1 to 100%, preferably 3 to 90%, more preferably 4 to 60%. Furthermore, the number of glutamic acids (k) is about 1 to 200, preferably about 1 to 90, particularly preferably about 3 to 60. The proportion of the bound combretastatins can be changed, for example, according to the amounts a block copolymer and combretastatins fed to the reaction for their binding reaction, and can be determined by an analysis of the reaction liquid.

For each of the constituent units of the glutamic acid structure in the high-molecular weight conjugate of combretastatins represented by the above general formula (II), the linking order is not limited, and may be of block type or random type.

In the general formula (I) or (II), t is an integer of about 5 to 11500, preferably an integer of about 8 to 2300, more preferably an integer of about 100 to 300.

The high-molecular weight conjugate of combretastatins of the present invention may form micelles with the polyethylene glycol moiety as an outer shell in water.

The high-molecular weight conjugate of combretastatins of the present invention comprising a block copolymer of a polyethylene glycol moiety and a polymer moiety having two or more carboxylic acid groups is obtained, for example, by linking a carboxylic acid group of the polymer moiety to a hydroxyl group of combretastatins via an ester bond using a dehydrating condensing agent in an organic solvent, and the present invention also include the manufacturing method; that is, a method of subjecting, for example, a block copolymer of a polyethylene glycol moiety-polyaspartic acid prepared according to the method described in Patent Document 5, or a block copolymer of a polyethylene glycol moiety-polyglutamic acid prepared according to the method described in Japanese Patent Application Laid-Open (KOKAI) No. 5-955, and combretastatins in which functional groups other than the groups to be reacted are protected if necessary, to a condensation reaction at 0 to 180°C, preferably 5 to 50°C, using a dehydrating condensing agent in an organic solvent in which two substances are dissolved. Furthermore, a reaction aid may also be used during the condensation reaction. The organic solvent used in the condensation reaction is not particularly limited, but preferred is an aprotic polar solvent such as N,N-dimethylformamide (DMF), 1,3-dimethyl-2-imidazolidinone (DMI) or N-methylpyrrolidone (NMP). As the dehydrating condensing agent, dicyclohexylcarbodiimide (DCC), diisopropylcarbodiimide (DIPC), 1-ethyl-3-(3-dimethylaminopropyl)carbodiimide hydrochloride (WSC), 1-ethoxycarbonyl-2-ethoxy-1,2-dihydroxyquinolinone (EEDQ) or the like can be used. As for the reaction aid, N,N-dimethylaminopyridine (DMAP) or the like can be used. After condensation reaction, conventional operations such as separation and purification and the like may be carried out to obtain the high-molecular weight conjugate of combretastatins. If necessary, deprotection may be carried out. For example, in the case of combretastatin AC-7700 (V), after protecting the amino group with a protecting group such as a t-butoxycarbonyl group and subjecting the resultant compound to the condensation reaction with a block copolymer, the compound of the present invention can be obtained by deprotection with trifluoroacetic acid or the like.

Furthermore, the high-molecular weight conjugate of combretastatins in which R5 is the group -N(R6)CONH(R7) group wherein R6 and R7, which may be identical or different, are each a (C3-C6) cyclic alkyl group, or a (C1-C5) alkyl group which may be substituted with a tertiary amino group, may be obtained also by a reaction using the aforementioned carbodiimides as a condensing agent.

As a method for manufacturing a compound in which R5 in the compound of general formula (I) or (II) is a (C1-C30) alkoxy group, a (C1-C30) aralkyloxy group, a (C1-C30) alkylamino group, a di(C1-C30) alkylamino group or an amino acid with a protected carboxyl group, there may be mentioned a method in which the carboxylic acid group of the polymer is first activated and then reacted with a corresponding alcohol or a corresponding amine, or an amino acid with a protected carboxyl group and the like in an amount desired to be linked under a basic condition; a method in which a corresponding alcohol, a corresponding amine or an amino acid with a protected carboxyl group or the like is first activated and then subjected to the condensation reaction with the polymer; and the like. After purification of the polymer, it is possible to re-activate unreacted carboxylic acid groups in the polymer by the same reaction, and hydroxyl groups of combretastatins may be condensed with the re-activated carboxylic acid groups. Alternatively, other alcohols, amines and the like may be repeatedly reacted to synthesize a mixture of polymers in which R5 is substituted with various substituents, to which hydroxyl groups of the combretastatins may subsequently be condensed therewith. Furthermore, after condensation of the combretastatins, a (C1-C30) alkoxy group, a (C1-C30) aralkyloxy group, a (C1-C30) alkylamino group, a di(C1-C30) alkylamino group, an amino acid with a protected carboxyl group or the like may be introduced.

The method for manufacturing the high-molecular weight conjugate of combretastatins of the present invention is not limited to the aforementioned methods.

The present invention also includes an anticancer agent comprising the high-molecular weight conjugate of combretastatins of the present invention as an active ingredient. Furthermore, the present invention also includes a vascular targeting agent comprising the high-molecular weight conjugate of combretastatins of the present invention as an active ingredient, that is, a therapeutic agent for a disease associated with neovascularization by controlling the blood flow, for example, a therapeutic agent for rheumatoid arthritis, age-related macular degeneration, diabetic retinopathy and the like. The high-molecular weight conjugate can be used in a dosage form which is conventionally used, including, for example, injections, tablets, powders and the like. Pharmaceutically acceptable carriers conventionally used in formulation, for example, binding agents, lubricants, disintegrants, solvents, excipients, solubilizing agents, dispersants, stabilizers, suspending agents, preservatives, soothing agents, colorants, flavors and the like can be used. Among others, the use as an injection is preferred, and usually, for example, water, physiological saline, a 5% glucose or mannitol solution, water-soluble organic solvents (for example, glycerol, ethanol, dimethylsulfoxide, N-methylpyrrolidone, polyethylene glycol, cremophor or a mixed liquid thereof), or a mixed liquid of water and the water-soluble organic solvent, and the like are used.

The dosage of the high-molecular weight conjugate of combretastatins of the present invention can vary, as a matter of course, with the sex, age, physiological conditions, pathological conditions and the like of the patient, and the high-molecular weight conjugate of combretastatins is usually administered parenterally at a dose of 0.01 to 500 mg/m², preferably 0.1 to 250 mg/m² as the active ingredient per day for an adult. Administration by injection is performed intravenously, intra-arterially, in the affected site (tumor site) and the like.

### EXAMPLES

Hereinafter, the present invention will be illustrated more specifically by with reference to Examples, but the present invention is not intended to be limited to these Examples.

### Example 1: Synthesis of compound 1 (conjugate of combretastatin A-4 and a block copolymer consisting of a methoxypolyethylene glycol moiety having a molecular weight of 5000 and a polyaspartic acid moiety having a polymerization number of 30: in general formula (I), R1 = Me (methyl group), R2 = trimethylene group, R3 = Ac (acetyl group), R4 = combretastatin A-4 residue, R5 = isopropylaminocarbonylisopropylamino group, d+e+f+g+h+i+j = 30, t = 113)

A methoxypolyethylene glycol-polyaspartic acid block copolymer (aspartic acid moiety: mixture of α-type and β-type, polymerization number: 30, 2670 mg) prepared according to the method described in Patent Document 5, and combretastatin A-4 (600 mg) synthesized by the method described in Non-Patent Document 1 were dissolved in DMF (60 ml), and DMAP (174 mg) and DIPC (2.97 ml) were added thereto. The mixture was stirred for 20 hours at 25°C. Ethyl acetate (180 ml) and diisopropyl ether (720 ml) were added to the reaction liquid, and was stirred for 30 minutes at room temperature. Subsequently, the precipitate was collected by filtration, and washed with ethyl acetate/diisopropyl ether (1/4 (v/v), 30 ml). The resultant precipitate was dissolved in acetonitrile/water (1/1 (v/v), 100 ml), and then the solution was passed through a column of ion-exchange resin (Dowex 50(H⁺) manufactured by Dow Chemical Company, 15 ml), and eluted with acetonitrile/water (1/1 (v/v), 20 ml). Water (60 ml) was added to the eluted fraction thus obtained, and then acetonitrile was distilled off under reduced pressure. Then, the residue was freeze-dried to obtain compound 1 (2670 mg).

On the basis of the amount of unreacted combretastatin A-4 in the reaction liquid determined by HPLC [high performance liquid chromatography: column; Inertsil ODS-3 (GL Sciences, Inc.), solvent system; 0.1% aqueous phosphoric acid solution-acetonitrile (50%-50% (v/v))], the content of combretastatin A-4 in compound 1 was determined as 10.4% (w/w), and the proportion of d+e based on d+e+f+g+h+i+j was determined as 10.6%. In compound 1, free combretastatin A-4 was not detected.

Furthermore, an isopropylaminocarbonylisopropylamino group can be introduced as R5 by this method, and the abundance ratio of the group is determined by ¹H-NMR (hydrogen nuclear magnetic resonance spectroscopy) of a solution obtained by dissolving the compound 1 in sodium deuteroxide/deuterium oxide/deuterated acetonitrile. The proportion of the isopropylaminocarbonylisopropylamino group based on the polyaspartic acid in compound 1, that is, the proportion of f+g based on d+e+f+g+h+i+j was 16.2%. The remaining aspartic acids are in the form of free carboxylic acid (h+i) or a cyclic structure (j).

### Example 2: Synthesis of compound 2 (conjugate of combretastatin A-4 and a block copolymer consisting of a methoxypolyethylene glycol moiety having a molecular weight of 12000 and a polyglutamic acid moiety having a polymerization number of 23: in general formula (II), R1 = Me (methyl group), R2 = trimethylene group, R3 = Ac (acetyl group), R4 = combretastatin A-4 residue, R5 = isopropylaminocarbonylisopropylamino group, k+m+n = 23, t = 273)

A methoxypolyethylene glycol-polyglutamic acid block copolymer (581 mg) prepared according to the method described in Japanese Patent Application Laid-Open (KOKAI) No. 5-955, and combretastatin A-4 (100 mg) synthesized by the method described in Non-Patent Document 1 were dissolved in DMF (4.5 ml), and DMAP (16.5 mg) and DIPC (0.283 ml) were added thereto and was stirred for 40 hours at 20°C. DIPC (0.070 ml) was added to the reaction liquid, and after the temperature reached 25°C, further stirring was continued for 1.5 hours. Ethanol (60 ml) and diisopropyl ether (240 ml) were added to the reaction liquid, and the mixture was stirred for 3 hours at room temperature. Subsequently, the precipitate was collected by filtration, and washed with ethanol/diisopropyl ether (1/4 (v/v), 50 ml). The resultant precipitate was dissolved in acetonitrile/water (1/1 (v/v), 50 ml), and then the solution was passed through a column of ion-exchange resin (Dowex 50 (H⁺) manufactured by Dow Chemical Company, 15 ml), and eluted with acetonitrile/water (1/1 (v/v), 50 ml). Water (3 ml) was added to the eluted fraction thus obtained, and then acetonitrile was distilled off under reduced pressure, compound 2 (550 mg) was then obtained by freeze-drying.

On the basis of the amount of unreacted combretastatin A-4 in the reaction liquid determined by HPLC, the content of combretastatin A-4 content in the compound 2 was determined as 11.2% (w/w). In compound 2, free combretastatin A-4 was not detected.

According to this method, an isopropylaminocarbonylisopropylamino group can be introduced as R5, and the abundance ratio of the group is determined by ¹H-NMR (hydrogen nuclear magnetic resonance spectroscopy) using compound 2 dissolved in sodium deuteroxide/deuterium oxide/deuterated acetonitrile. The proportion of the isopropylaminocarbonylisopropylamino group based on the polyaspartic acid, that is, the proportion of m based on k+m+n was 32%.

### Example 3: Synthesis of compound 3 (conjugate of combretastatin A-4 and a block copolymer consisting of a methoxypolyethylene glycol moiety having a molecular weight of 12000 and a polyaspartic acid moiety having a polymerization number of 33: in general formula (I), R1 = Me (methyl group), R2 = trimethylene group, R3 = Ac (acetyl group), R4 = combretastatin residue, R5 = isopropylaminocarbonylisopropylamino group and O-benzyl-phenylalanyl group, d+e+f+g+h+i+j = 33, t = 273)

A methoxypolyethylene glycol-polyaspartic acid block copolymer (aspartic acid moiety: a mixture of α-type and β-type, polymerization number of aspartic acid: 33, 605.4 mg) prepared according to the method described in Patent Document 5, and combretastatin A-4 (100 mg) prepared by the method described in WO 02/06279 were dissolved in DMF (8.5 ml), and phenylalanine benzyl ester hydrochloride (83.4 mg), triethylamine (0.04 ml), DMAP (16 mg) and DIPC (0.4 ml) were added thereto and was stirred for 20 hours at 15°C, and then further stirred for 4 hours at 25°C. Ethyl acetate (70 ml) and heptane (70 ml) were added to the reaction liquid, and was stirred for 30 minutes at room temperature. Subsequently, the precipitate was collected by filtration, and washed with ethyl acetate/heptane (1/1 (v/v), 20 ml). The resultant precipitate was dissolved in acetonitrile/water (1/1 (v/v), 20 ml), and then the solution was passed through a column of ion-exchange resin (Dowex 50 (H⁺) manufactured by Dow Chemical Company, 3 ml), and eluted with acetonitrile/water (1/1 (v/v), 20 ml). Water (35 ml) was added to the eluted fraction thus obtained, and then acetonitrile was distilled off under reduced pressure. Compound 3 (710 mg) was then obtained by freeze-drying.

On the basis of the amount of unreacted combretastatin A-4 in the reaction liquid determined by HPLC (high performance liquid chromatography), the content of combretastatin A-4 in the compound 3 was determined as 7.9% (w/w), and the proportion of d+e based on d+e+f+g+h+i+j was 14%. Free combretastatin A-4 in the compound 3 was not detected.

The O-benzyl-phenylalanyl group introduced as one of R5 was determined by quantifying the amount of benzyl alcohol released by hydrolyzing compound 3 in an aqueous solution of acetonitrile-sodium hydroxide at 40°C for 6 hours, and subjected to elution. The proportion of the O-benzyl-phenylalanyl group based on the polyaspartic acid, that is, the proportion of O-benzyl-phenylalanyl groups bound to f+g based on d+e+f+g+h+i+j was 27%. According to this method, an isopropylaminocarbonylisopropylamino group is also introduced as R5, and the abundance ratio of the group is determined by ¹H-NMR (hydrogen nuclear magnetic resonance spectroscopy) using compound 3 dissolved in sodium deuteroxide/ deuterium oxide/ deuterated acetonitrile. The proportion of the isopropylaminocarbonylisopropylamino group based on the polyaspartic acid, that is, the proportion of f+g based on d+e+f+g+h+i+j was 15% with regard to the product having isopropylaminocarbonylisopropylamino groups bound. As a result, the proportion of the total amount of R5 to the polyaspartic acid, that is, the proportion of f+g based on d+e+f+g+h+i+j was 42%. The remaining aspartic acids were in the form of free carboxylic acid (h+i) or a cyclic structure (j).

### Test Example 1: Release of drug in the absence of enzyme

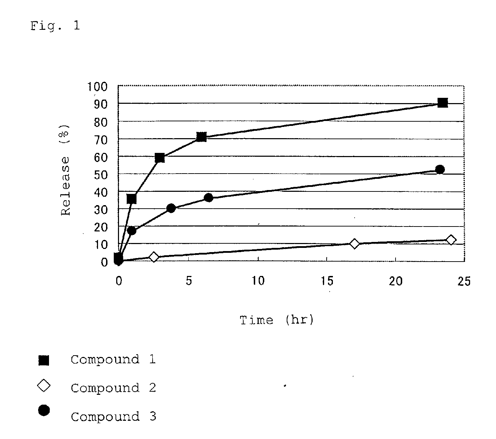

Compound 1, compound 2 or compound 3 was dissolved in PBS (phosphate buffered physiological saline; pH 7.1) to a polymer concentration of 1 mg/ml, and incubated at 37°C. Combretastatin A-4 released from the high-molecular weight conjugate was separated and quantified by HPLC using a standard curve. The percentage of the quantified value based on the total drug amount determined from the drug content of the high-molecular weight conjugate is shown in Fig. 1.

As is obvious from Fig. 1, the high-molecular weight conjugates of the present invention (compound 1, compound 2 and compound 3) significantly released combretastatin A-4 even in the absence of hydrolyzing enzymes. In particular, compound 1 and compound 3 which have a succinic acid monoamide moiety released combretastatin A-4 more rapidly, compared with the compound 2 which does not have a succinic acid monoamide moiety. The results shown in Fig. 1 demonstrates the excellent drug release performance of the high-molecular weight conjugate of the present invention in the absence of enzymes.

### Test Example 2: Antitumor effect

Mouse colon cancer, Colon26, maintained by serial subcutaneous subculture in mice was minced into about 2-mm square fragments, and the fragments were subcutaneously transplanted on the dorsal part of female CDF1 mice with a trocar. Seven days after tumor transplantation, the high-molecular weight conjugate of the present invention (compound 1) or a control drug (combretastatin A-4) was administered a single time intravenously to the mouse tail vein, at a dose respectively calculated on the basis of combretastatin A-4. The control is a group not given the drug. Compound 1 was dissolved in a 5% glucose injection solution and used. Combretastatin A-4 was dissolved in dimethylsulfoxide and Cremophor EL (manufactured by Sigma-Aldrich Company), and was used after diluted with a 5% glucose injection solution at the time of use. After the administration, the major axis (L mm) and the minor axis (W mm) of the tumor were measured using a caliper, and the volume of tumor was calculated by the formula: (L × W²)/2. Table 1 shows the relative tumor volume based on the tumor volume on the day of initiation of administration.

**[Table 1]**

| | Dose | Days after administration (days) | | | | |
|---|---|---|---|---|---|---|
| | | 0 | 2 | 4 | 7 | 8 |
| Compound 1 | 200 mg/kg | 1.00 | 0.87 | 0.98 | 1.15 | 1.71 |
| | 100 mg/kg | 1.00 | 0.99 | 1.38 | 2.87 | 4.91 |
| Combretastatin A-4 | 400 mg/kg | 1.00 | 1.24 | 2.21 | 5.09 | 6.41 |
| | 200 mg/kg | 1.00 | 1.77 | 3.73 | 9.25 | 12.03 |
| Control | | 1.00 | 2.70 | 5.02 | 10.51 | 11.59 |

Table 1 clearly revealed that the high-molecular weight conjugate of the present invention (compound 1) has superior antitumor activity to combretastatin A-4 at a dose lower than that of combretastatin A-4.

### Test Example 3: Antitumor effect

Mouse colon cancer, Colon26, maintained by serial subcutaneous subculture in mice was minced into about 1-mm square fragments, and the fragments were subcutaneously transplanted on the dorsal part of female CDF1 mice with a trocar. Seven days after tumor transplantation, the high-molecular weight conjugate of the present invention (compound 2 and compound 3) or a control drug (combretastatin A-4 phosphoric acid ester) synthesized by a method according to Non-Patent Document 1 was administered a single time intravenously to the mouse tail vein, at a dose respectively calculated on the basis of combretastatin A-4. The control is a group not given the drug. Compound 2, compound 3 and the control drug were all dissolved in a 5% glucose injection solution and used. After the administration, the major axis (L mm) and the minor axis (W mm) of the tumor were measured using a caliper, and the volume of tumor was calculated by the formula: (L × W²)/2. Table 2 shows the relative tumor volume based on the tumor volume on the day of initiation of administration.

**[Table 2]**

| | Dose | Days after administration | | | | |
|---|---|---|---|---|---|---|
| | | 0 | 2 | 4 | 7 | 9 |
| Compound 2 | 50 mg/kg | 1.00 | 0.99 | 1.11 | 3.05 | 5.52 |
| Compound 3 | 100 mg/kg | 1.00 | 0.79 | 0.99 | 0.84 | 1.18 |
| | 50 mg/kg | 1.00 | 1.15 | 1.20 | 1.59 | 3.87 |
| Combretastatin A-4 phosphoric acid ester | 200 mg/kg | 1.00 | 1.57 | 3.12 | 9.33 | 10.11 |
| Control | | 1.00 | 2.82 | 5.70 | 12.23 | 19.58 |

From the Table 2, it is obvious that the high-molecular weight conjugate of the present invention (compound 2 and compound 3) have superior antitumor activity to combretastatin A-4 phosphoric acid ester at a lower dose.

### Brief Description of the Drawings

Fig. 1 shows the percentage of the amount of combretastatin A-4 released in PBS solutions (pH 7.1, 37°C) from compound 1 and 3 of Example 1 of the present invention (conjugate of (methoxypolyethylene glycol derivative-polyaspartic acid)-combretastatin A-4) and compound 2 of Example 2 (conjugate of (methoxypolyethylene glycol derivative-polyglutamic acid)-combretastatin A-4), based on the total amount of the bound drug.

## Claims

1. A high-molecular weight conjugate of combretastatins, comprising a block copolymer of a polyethylene glycol moiety and a polymer moiety having two or more carboxylic acid groups, in which a carboxylic acid group of the polymer moiety is linked to a hydroxyl group of combretastatins via an ester bond.

2. The high-molecular weight conjugate of combretastatins according to claim 1, wherein the polymer moiety having carboxylic acid groups is a polymer having succinic acid monoamide moieties.

3. The high-molecular weight conjugate of combretastatins according to claim 2, wherein the polymer having succinic acid monoamide moieties is polyaspartic acid.

4. The high-molecular weight conjugate of combretastatins according to any one of claims 1 to 3, which is a compound represented by general formula (I): 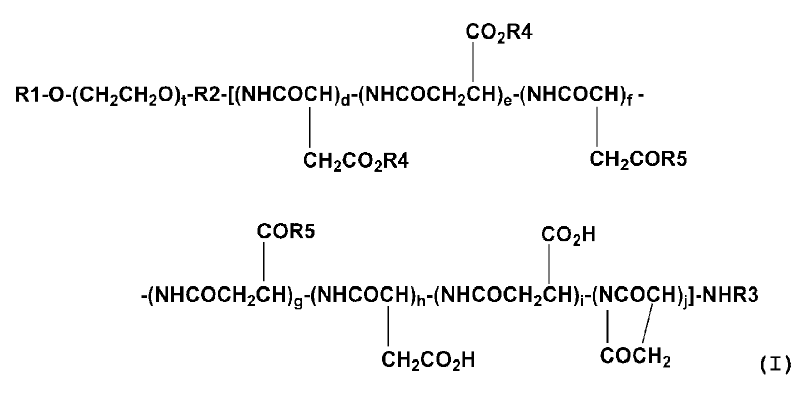 wherein R1 represents a hydrogen atom or a (C1-C6) alkyl group; R2 represents a linking group; R3 represents a hydrogen atom or a (C1-C6) acyl group; R4 represents a residue of a hydroxyl group of the combretastatins; R5 represents a group selected from the group consisting of a (C1-C30) alkoxy group, a (C7-C30) aralkyloxy group, a (C1-C30) alkylamino group, a di(C1-C30) alkylamino group, an amino acid with a protected carboxyl group and -N(R6)CONH(R7) wherein R6 and R7, which may be identical or different, each represent a (C3-C6) cyclic alkyl group, or a (C1-C5) alkyl group which may be substituted with a tertiary amino group; t represents an integer from 5 to 11500; d, e, f, g, h, i or j each independently represent an integer from 0 to 200; with the proviso that d+e represent an integer from 1 to 200; and d+e+f+g+h+i+j represent an integer from 3 to 200; and the respective constituent units of the polyaspartic acid are linked in any order.

5. The high-molecular weight conjugate of combretastatins according to claim 4, wherein R1 is a (C1-C6) alkyl group; R2 is a (C2-C6) alkylene group; R3 is a (C1-C6) acyl group; t is an integer from 8 to 2300; and d, e, f, g, h, i or j are each independently an integer from 0 to 100; with the proviso that d+e is an integer from 1 to 100, and d+e+f+g+h+i+j is an integer from 6 to 100.

6. The high-molecular weight conjugate of combretastatins according to claim 4 or 5, wherein R1 is a (C1-C3) alkyl group; R2 is a (C2-C4) alkylene group; R3 is a (C1-C3) acyl group; t is an integer from 100 to 300; and d, e, f, g, h, i or j each independently is an integer from 0 to 90; with the proviso that d+e is an integer from 1 to 90, and d+e+f+g+h+i+j is an integer from 15 to 90.

7. The high-molecular weight conjugate of combretastatins according to claim 1, wherein the polymer moiety having carboxylic acid groups is polyglutamic acid.

8. The high-molecular weight conjugate of combretastatins according to claim 1 or 7, which is a compound represented by general formula (II): 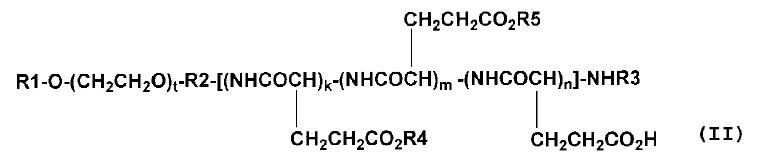 wherein R1 represents a hydrogen atom or a (C1-C6) alkyl group; R2 represents a linking group; R3 represents a hydrogen atom or a (C1-C6) acyl group; R4 represents a residue of a hydroxyl group of the combretastatins; R5 represents a group selected from the group consisting of a (C1-C30) alkoxy group, a (C7-C30) aralkyloxy group, a (C1-C30) alkylamino group, a di(C1-C30) alkylamino group, an amino acid with a protected carboxyl group and -N(R6)CONH(R7); R6 and R7, which may be identical or different, each represent a (C3-C6) cyclic alkyl group or a (C1-C5) alkyl group which may be substituted with a tertiary amino group; t represents an integer from 5 to 11500; k represents an integer from 1 to 200; m and n each independently represent an integer from 0 to 200; with the proviso that k+m+n represents an integer from 3 to 200; and the respective constituent units of the polyglutamic acid are linked in any order.

9. The high-molecular weight conjugate of combretastatins according to claim 8, wherein R1 is a (C1-C6) alkyl group; R2 is a (C2-C6) alkylene group; R3 is a (C1-C6) acyl group; t is an integer from 8 to 2300; and k is an integer from 1 to 90; and m and n are each independently an integer from 0 to 90; with the proviso that k+m+n is an integer from 6 to 90.

10. The high-molecular weight conjugate of combretastatins according to claim 8 or 9, wherein R1 is a (C1-C3) alkyl group; R2 is a (C2-C4) alkylene group; R3 is a (C1-C3) acyl group; t is an integer from 100 to 300; k is an integer from 3 to 60; and m and n are each independently an integer from 0 to 60; with the proviso that k+m+n is an integer from 6 to 60.

11. The high-molecular weight conjugate of combretastatins according to any one of claims 1 to 10, wherein the combretastatin is combretastatin A-4.

12. A high-molecular weight conjugate of combretastatins, obtained by, in a block copolymer of a polyethylene glycol moiety and a polymer moiety having two or more carboxylic acid groups, linking a carboxylic acid group of the polymer moiety to a hydroxyl group of the combretastatins via an ester bond using a dehydrating condensing agent in an organic solvent.

13. A method for manufacturing a high-molecular weight conjugate of combretastatins according to any one of claims 1 to 11, the method comprising linking a carboxylic acid group of the polymer moiety to a hydroxyl group of the combretastatins via an ester bond using a dehydrating condensing agent in an organic solvent.

14. An anticancer agent comprising the high-molecular weight conjugate of combretastatins according to claims 1 to 12, as an active ingredient.

15. A vascular targeting agent comprising the high-molecular weight conjugate of combretastatins according to claims 1 to 12, as an active ingredient.
